(19) Europäisches Patentamt
European Patent Office
Office européen des brevets

(11) Publication number : 0 610 154 A1

(12) # EUROPEAN PATENT APPLICATION

(21) Application number : 94810042.5

(51) Int. Cl.$^5$ : D21H 21/30, D21H 19/46

(22) Date of filing : 25.01.94

(30) Priority : 02.02.93 GB 9302024

(43) Date of publication of application :
10.08.94 Bulletin 94/32

(84) Designated Contracting States :
AT BE CH DE ES FR GB IT LI NL SE

(71) Applicant : CIBA-GEIGY AG
Klybeckstrasse 141
CH-4002 Basel (CH)

(72) Inventor : Rohringer, Peter
Sechsjuchartenstrasse 1
CH-4124 Schönenbuch (CH)
Inventor : Fletcher, Ian John
Bürgenstal 27
CH-4312 Magden (CH)

(54) **Fluorescent whitening of paper.**

(57) A method for the bleed-free fluorescent whitening of a paper surface is provided comprising contacting the paper surface with an aqueous coating composition comprising a coumarin-based fluorescent whitening agent. An aqueous coating composition comprising a binder, a coumarin-based fluorescent whitening agent and optionally an inorganic or organic pigment is also provided.

EP 0 610 154 A1

The present invention relates to a method for the fluorescent whitening of paper surfaces using coumarin fluorescent whitening agents.

An increasingly important parameter, which determines whether or not a particular fluorescent whitening agent can be used in paper applications, is the bleed fastness of the whitening agent. This parameter is important e.g. because of toxicological considerations (e.g. in relation to the packaging of foodstuffs) and because of technical requirements (e.g. for use in photopapers).

The stilbene class of fluorescent whitening agents has been very widely used in the paper industry, but this whitener class has inadequate bleed fastness to water.

In DE-A-2,242,597 preparations are described for the whitening of coating compositions for paper. The preparations contain a dispersion whitener in a colourless organic liquid which is immiscible with water and has a boiling point above 150°C. Among a wide range of dispersion whiteners, there are mentioned certain coumarin fluorescent whitening agents.

Surprisingly, it has now been found that the coumarin class of fluorescent whitening agents, when used in aqueous coating compositions to whiten paper surfaces, exhibits an excellent bleed fastness to water, which bleed fastness is considerably better than that of stilbene optical whitening agents, while avoiding the problems associated with the use of high-boiling organic solvents.

Accordingly, the present invention provides a method for the fluorescent whitening of a paper surface, comprising contacting the paper surface with an aqueous coating composition containing a coumarin-based fluorescent whitening agent.

The coumarin-based fluorescent whitening agent preferably has the formula:

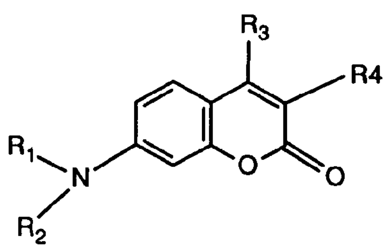 (1)

in which $R_1$ and $R_2$, independently, are hydrogen or $C_1$-$C_4$alkyl or $R_1$ and $R_2$, together with the nitrogen atom to which they are each attached, are the atoms required to complete a pyrazole or triazine ring; $R_3$ is hydrogen or $C_1$-$C_4$alkyl; and $R_4$ is hydrogen or a residue having the formula:

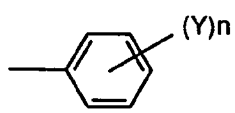

in which n is 1 or 2 and Y is hydrogen, $C_1$-$C_4$alkyl, halogen, preferably bromine or, especially, chlorine, $SO_3H$, $SO_2NH_2$, $SO_2NH(C_1$-$C_4alkyl)$, $SO_2N(C_1$-$C_4alkyl)_2$, $SO_2NHCH_2CH_2OH$, $SO_2NHCH_2CH_2O(C_1$-$C_4alkyl)$ or $SO_2NHCH_2CH_2CH_2N(C_1$-$C_4alkyl)_2$.

The compounds of formula (1) are known and have been disclosed, together with their production, e.g. in British Patent Specification No. 655,258, U.S. Patent Specification No. 3,123,617 and U.S. Patent Specification No. 2,945,033.

One preferred class of compounds of formula (1) is that having the formula:

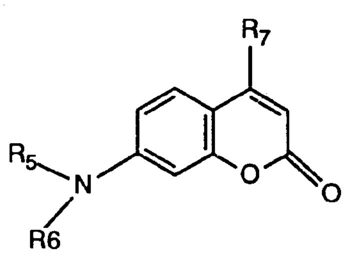 (2)

in which $R_5$ and $R_5$, independently, are $C_1$-$C_4$alkyl, preferably ethyl, and $R_7$ is $C_1$-$C_4$alkyl, preferably methyl.

The compounds of formula (2) are known and have been disclosed, together with their production, e.g. in British Patent Specification No. 655,258, in which they are described as intermediates for sulphonated compounds useful as fluorescent whitening agents, especially on animal fibres. Their use in aqueous coating compositions for whitening paper is not described.

A further preferred class of compounds of formula (1) is that comprising 7-pyrazolyl-1-yl-3-phenyl-coumarins having the formula:

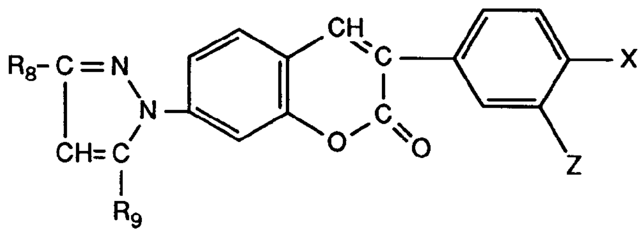

(3)

in which $R_5$ is hydrogen, $C_1$-$C_4$alkyl, phenyl or benzyl; $R_9$ is hydrogen, $C_1$-$C_4$alkyl or phenyl; X is hydrogen, chlorine or $C_1$-$C_4$alkyl; and Z is hydrogen, $-SO_3H$, $-SO_2NH_2$, $-SO_2NH-CH_3$, $-SO_2-NH-CH_2CH_3$, $-SO_2-NH-CH_2CH_2OH$, $-SO_2-NH-CH_2CH_2OCH_3$, $-SO_2-NH-CH_2CH_2-OCH_2CH_3$, $-SO_2-NH-CH_2CH_2N(CH_2CH_3)_2$, $-SO_2-NH-CH_2CH_2CH_2N(CH_3)_2$, $-SO_2-N(CH_3)_2$ or $-SO_2N(CH_2CH_3)_2$.

Preferably, $R_5$ is hydrogen, methyl or ethyl; $R_9$ is hydrogen or methyl; X is hydrogen, methyl or chlorine; and Z is hydrogen.

The compounds of formula (3), and their production are described in U.S. Patent Specification No. 3,123,617, in which they are said to be useful as optical brightening agents for synthetic polyester fibres. Their use in the fluorescent whitening of paper surfaces is neither mentioned nor suggested.

A particularly preferred group of coumarin-based fluorescent whitening agents, for use in the method of the present invention, is that group of coumarin fluorescent brighteners having the formula:

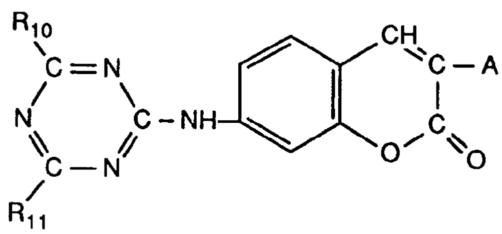

(4)

in which $R_{10}$ is amino, $C_1$-$C_4$alkylamino, di($C_1$-$C_4$alkyl)amino, cyclohexylamino, monoethanolamino, diethanolamino, N-phenylamino, morpholino, piperidino, Cl, Br, hydroxy, $C_1$-$C_4$alkoxy, phenoxy, $C_1$-$C_4$alkylphenoxy, chlorophenoxy, phenylthio, chlorophenylthio, hydroxycyclohexylamino, $C_1$-$C_4$alkylamino-$C_1$-$C_4$alkylamino, N-$C_1$-$C_4$alkylphenylamino, N-$C_1$-$C_4$alkoxyphenylamino, N-chlorophenylamino, benzylamino, N-pyridylamino, N-thiazolylamino, N-dihydro-imidazolylamino, N-triazolylamino or N-tetrazolylamino; $R_{11}$ is amino, $C_1$-$C_4$alkylamino, di-($C_1$-$C_4$-alkyl)amino, cyclohexylamino, monoethanolamino, diethanolamino, N-phenylamino, morpholino or piperidino; and A is phenyl, chlorophenyl, methylphenyl or dimethylphenyl.

Preferably $R_{10}$ is amino, diethylamino, chloro, morpholino, monoethanolamino or N-ethyl-N-butylamino; and $R_{11}$ is preferably diethylamino, monoethanolamino, morpholino, N-ethyl-N-butylamino or phenylthio.

Of especial interest, within the class of compounds of formula (4) is the compound of formula:

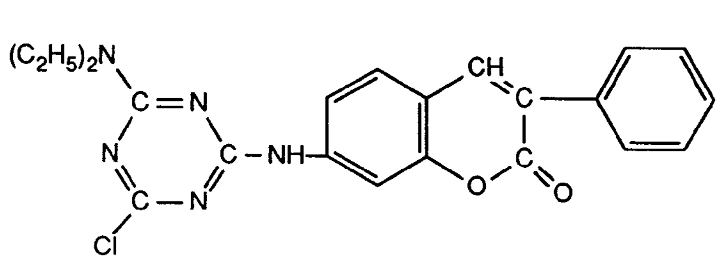

(5)

The compounds of formula (4), and their production are described in U.S. Patent Specification No. 2,945,033 in which they are described as optical brighteners for synthetic materials such as vinyl polymers or textiles. Their use in dyeing paper surfaces is neither disclosed nor suggested.

The fluorescent brightener, for use in the aqueous coating composition used according to the method of the present invention, is usually formulated as a dispersion or emulsion and thus contains, in addition to the coumarin-based fluorescent whitening agent and water, at least one dispersing agent and/or emulsifier and optionally other auxiliaries.

The fluorescent brightener formulation used, preferably contains customary anionic or cationic and/or non-ionic emulsifiers and/or dispersing agents as the dispering agents and/or emulsifiers, preferably in amounts of 2-20 %, in particular 5-10 %, based on the weight of fluorescent brightener.

Examples of anionic emulsifiers which may be mentioned are:

Carboxylic acids and their salts, such as the sodium, potassium or ammonium salts of lauric, stearic or oleic acid, acylation products of aminocarboxylic acids and their salts, for example the sodium salt of oleoylsarcoside, sulfates, such as fatty alcohol sulfates, for example lauryl sulfate and coconut sulfate, sulfates of hydroxy fatty acid esters, for example sulfated castor oil, and of fatty acid hydroxylkylamides, for example sulfated coconut oil acid ethanolamide, and sulfates of partially esterified or etherified polyhydroxy compounds such as sulfated oleic acid monoglyceride or glycerol ethersulfates, and furthermore sulfates of substituted polyglycol ethers, for example nonylphenyl polyglycol ether sulfate, sulfonates, such as primary and secondary alkylsulfonates, for example $C_{12}$-$C_{16}$paraffinsulfonic acids and sodium salts thereof, alkylsulfonates with acyl radicals bonded in amide or ester form, such as oleylmethyl-tauride, and sulfonates of polycarboxylic acid esters, such as diisooctylsulfato-succinic acid esters; and furthermore those with aromatic groups such as alkylbenzene, for example dodecylbenzene-, alkylnaphthalene-, such as dibutylnaphthlene, and alkylbenzimidazole, such as tetradecylbenzimidazole-sulfonates.

Examples of non-ionic emulsifiers which may be mentioned are:

Esters and ethers of polyalcohols, such as alkyl polyglycol ethers, for example lauryl alcohol or oleyl alcohol, polyethylene glycol ethers, acyl polyglycol ethers, such as oleic acid polyglycol ether, alkylaryl polyglycol ethers, such as the ethoxylation products of nonyl- and dodecylphenol, acylated amino-alkanol polyglycol ethers, and furthermore the known non-ionic surfactants which are derived from fatty amines, such as stearylamine, fatty acid amides or sugars and derivatives thereof.

The anionic dispersing agents are the customary dispersing agents, for example condensation products of aromatic sulfonic acids with formaldehyde or ligninsulfonates, for example the compounds obtainable under the description of sulfite waste liquor. However, naphthalenesulfonic acid/formaldehyde condensation products and especially ditolyether sulfonic acid/formaldehyde condensation products are particularly suitable. Mixtures of these dispersing agents can also be used.

Non-ionic dispersing agents which may be mentioned are the ethylene oxide adducts of the class of addition products of ethylene oxide on higher fatty acids, saturated or unsaturated fatty alcohols, mercaptans, fatty acid amides, fatty acid alkylolamides or fatty amines or alkylphenols or alkylthiophenols having at least 7 carbon atoms in the alkyl radical, and furthermore ricinoleic acid esters or hydroxyabietyl alcohol. Some of the ethylene oxide units can be replaced by other epoxides, for example styrene oxide or, in particular, propylene oxide.

Ethylene oxide adducts which may be mentioned specifically are:

a) reaction products of saturated and/or unsaturated fatty alcohols having 8 to 20 C atoms with 20 to 100 mol of ethylene oxide per mol of alcohol;

b) reaction products of alkylphenols having 7 to 12 C atoms in the alkyl radical with 5 to 20 mol, preferably 8 to 15 mol, of ethylene oxide per mol of phenolic hydroxyl group;

c) reaction products of saturated and/or unsaturated fatty amines having 8 to 20 C atoms with 5 to 20 mol of ethylene oxide per mol of amine;

d) reaction products of saturated and/or unsaturated fatty acids having 8 to 20 C atoms with 5 to 20 mol of ethylene oxide per mol of fatty acid;

e) a reaction product of 1 mol of ricinoleic acid ester and 15 mol of ethylene oxide;

f) a reaction product of 1 mol of hydroxyabietyl alcohol and 25 mol of ethylene oxide;

Mixtures of the ethylene oxide adducts according to a) to f) with one another can also be used. These mixtures are obtained by mixing individual reaction products or directly by ethoxylation of a mixture of the compounds on which the adducts are based. An ethoxylated nonylphenyol is preferably used.

Possible cationic dispersing agents are, for example, quaternary fatty amine polyglycol ethers.

The fluorescent brightener formulation for use in producing the aqueous coating composition used according to the method of the present invention may also contain at least one specific water-insoluble aromatic or $C_{14}$-$C_{18}$ fatty alcohol auxiliary, or auxiliary mixture, which has a melting point between above 40°C and 200°C,

preferably up to 120°C. Cetyl alcohol and, in particular, compounds of the formula:

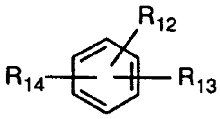

(6)

in which $R_{12}$ is $-COOR_{15}$, $-COR_{15}$, $-SO_2NHR_{15}$, $R_{15}$ or OH; $R_{13}$ is H, $-COOR_{15}$, $-COR_{15}$, $-SO_2NHR_{15}$ or $R_{15}$; $R_{14}$ is H or phenylene; and $R_{15}$ is $C_1$-$C_8$alkyl, $C_3$-$C_8$cycloalkyl or phenyl, each of which is optionally substituted by $C_1$-$C_8$alkyl, $C_3$-$C_8$cycloalkyl or phenyl.

Examples of such auxiliaries of formula (6) include benzylphenylketone, phenylsalicylate, benzophenone, dibenzyl, p-benzylbiphenyl, benzenesulfanilide, 1-hydroxy-2-phenylnaphthoate, diphenylterephthalate, dimethylterephthate and dicyclohexyl phthalate.

Such auxiliaries may be used in amounts of 0.1 to 150 parts, especially 1 to 150 parts, more especially in amounts of 9 to 100 parts, by weight per part of the fluorescent whitener.

The fluorescent brightener formulation for use in producing the coating composition , in addition, also contain 45-95 % of water and optionally preservatives and foam suppressants.

The fluorescent brightener formulation for use in preparing coating composition to be used according to the invention are obtained by simple mixing or dry grinding of the components, or by melting the dispersion fluorescent brightener and any auxiliary together, if appropriate in the presence of dispersing agents and/or emulsifiers, allowing the melt to solidify and then subjecting the product to dry grinding or if appropriate wet grinding, if appropriate in the presence of dispersing agents and/or emulsifiers.

The fluorescent brightener formulations so obtained are suitable for fluorescent brightening of aqueous coating compositions usually employed in the paper industry, and in particular for fluorescent brightening of non-pigmented, but especially pigmented, coating compositions. These known aqueous coating compositions contain as binders, inter alia, plastics dispersions based on copolymers of butadiene/styrene, acrylonitrile/butadiene/styrene, acrylic acid esters, ethylene/vinyl chloride and ethylene/vinyl acetate; or homopolymers, such as polyvinyl chloride, polyvinylidene chloride, polyethylene and polyvinyl acetate or polyurethanes. A preferred binder consists of styrene/butyl acrylate styrene/butadiene/ acrylic acid copolymer or styrene/butadiene rubbers. Other polymer latices are described, for example, in U.S. Patent Specifications 3,265,654, 3,657,174, 3,547,899 and 3,240,740. The fluorescent brightener formulation is incorporated into these binders, for example, by means of melt emulsification.

Aluminium silicates, such as China clay and kaolin, and furthermore barium sulfate, satin white, titanium dioxide or calcium carbonate (chalk) are usually employed for pigmenting the aqueous coating compositions.

Recipes for such known aqueous coating compositions for paper are described, for example, in J.P. Casey "Pulp and Paper"; Chemistry and Chemical Technology, 2nd edition, Volume III, pages 1684-1649 and in McGraw-Hill "Pulp and Paper Manufacture", 2nd and 5th edition, Volume II, page 497.

The aqueous coating compositions used according to the method of the present invention preferably contain 30 to 70 % by weight of a white pigment. The binder is preferably used in an amount which is sufficient to make the dry content of polymeric compound up to 1 to 30 % by weight, preferably 5 to 25 % by weight, of the white pigment. The amount of fluorescent brightener preparation used according to the invention is calculated so that the fluorescent brightener is present in amounts of 0.01 to 2 % by weight, in particular 0.1 to 1 % by weight, based on the white pigment.

The aqueous coating composition used in the method according to the invention can be prepared by mixing the components in any desired sequence at temperature from 10 to 100°C, preferably 20 to 80°C. The components here also include the customary auxiliaries which can be added to regulate the rheological properties, such as viscosity or water retention capacity, of the coating compositions. Such auxiliaries are, for example, natural binders, such as starch, casein, protein or gelatin, cellulose ethers, such as carboxyalkylcellulose or hydroxyalkylcellulose, alginic acid, alginates, polyethylene oxide or polyethylene oxide alkyl ethers, copolymers of ethylene oxide and propylene oxide, polyvinyl alcohol, polyvinylpyrrolidone, water-soluble condensation products of formaldehyde with urea or melamine, polyphosphates or polyacrylic acid salts.

The aqueous coating composition used according to the method of the present invention is used for coating paper or special papers such as cardboard or photographic papers.

The aqueous coating composition used according to the method of the present invention is novel *per se* and, as such, forms a further aspect of the present invention.

The coating composition used according to the method of the invention can be applied to the substrate by any conventional process, for example with an air blade, a coating blade, a brush, a roller, a doctor blade

or a rod, after which the coatings are dried at paper surface temperatures in the range from 70 to 200°C, preferably 90 to 130°C, to a residual moisture content of 3-8 %, for example with infra-red driers and/or hot-air driers. Comparably high degrees of whiteness are thus achieved even at low drying temperatures

By the use of the method according to the invention, the coatings obtained are distinguished by optimum distribution of the dispersion fluorescent brightener over the entire surface and an increase in the level of whiteness thereby achieved, a high fastness to light and excellent bleed-fastness to water.

The invention is illustrated in more detail by the following Examples, in which all the parts and percentages are expressed by weight, unless indicated otherwise.

Example 1:

A) Dispersion of the Fluorescent Brightener.

6 g of the fluorescent brightener having the formula:

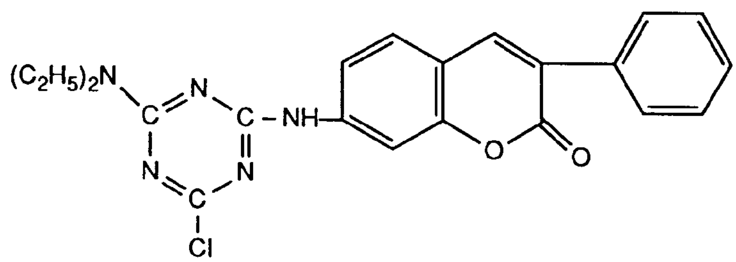

(5)

are ground with 1.5 g of a dispersing agent (condensation product of ditolylethersulfonic acid with formaldehyde-Baykanol SL) and 22.5 g water, over a period of 16 hours, in a glass bead mill. The resulting fine dispersion, containing 20 % fluorescent brightening agent, is then separated from the glass beads in a sieve.

B) Preparation of the Coating Composition

The following formulation is made up:

| | |
|---|---|
| 20 parts | of a commercial pigment (clay SPS) |
| 80 parts | of a commercial calcium carbonate (Hydrocarb 90) |
| 0,3 part | of a commercial 50 % solution of a polyacrylic acid |
| 18 parts | of a commercial 50 % dispersion of a styrene/butyl rubber latex (Dow Latex 955) |
| 0,5 part | of commercial carboxymethylcellulose |
| 0,5 part | of commercial polyvinylalcohol (Mowiol 4-98) |
| 0,8 part | of commercial 65 % melamine/formaldehyde precondensate (Protex M3M) |

To separate portions of the coating composition 0,05 %, 0,10 %, 0,20 %, 0,40 % or 0,80 % (based on total pigment-clay and carbonate) of the fluorescent whitener (active substance) under test is added.

After the addition of the fluorescent whitener, the content of dry substance in the coating colour is adjusted to 60 %. The pH is adjusted to 9.5 using NaOH.

C) The Paper Coating Application

Commercial base coated paper of LWC (light weight coated) quality having a weight per unit area of 39 g/m$^2$, a content of 50 % of mechanical wood pulp and a whiteness of $R_{457}$ = 70.9. (Reflection at 457 nm) is coated in a Dow laboratory coater. The drying is effected with hot air at a temperature of 195-200°C, until the moisture content is constant under standard conditions (about 7 % by weight). The coating weight, after acclimatization (23°C, 50 % relative humidity) is 12,5 ± 0,5 g/m$^2$.

D) Results

The results obtained are set out in the following Table 1:

Table 1

| % Content of Fluorescent Whitener (Active Substance) | | | | | | | | | | | |
|---|---|---|---|---|---|---|---|---|---|---|---|
| 0 | | 0.05 | | 0.10 | | 0.20 | | 0.40 | | 0.80 | |
| $R_{457}$ Whiteness Level | | | | | | | | | | | |
| 77.6 | | 81.7 | | 83.0 | | 85.2 | | 88.7 | | 88.6 | |
| Bleeding Fastness to Water after 10 min/24 h | | | | | | | | | | | |
| 5 | 5 | 4 | 3 | 5 | 3 | 5 | 4 | 5 | 4 | 5 | 4 |

Bleed Fastness is determined according to DIN 53991 - a mark of 5 corresponds to zero bleed, a mark of 1 denotes very severe bleeding.

Using the procedure described in Example 1, similar results are obtained when the compound of formula (5) is replaced by a compound of formula (4):

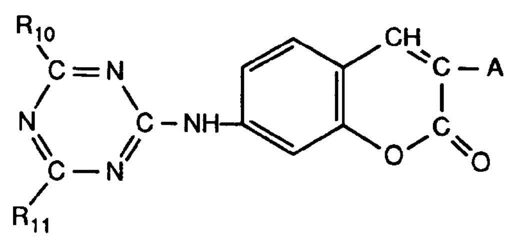

(4)

in which A is phenyl, $R_{10}$ is Cl and $R_{11}$ is as indicated below:

o-toluidino
m-methoxyanilino
p-chloranilino
o-ethoxyanilino
2-hydroxycyclohexylamino
β-phenylethylamino
monoethanolamino
diethanolamino
ethylamino
thiazolyl-(2)-amino
4,5-dihydro-imidazolyl-(2)-amino
pyridyl-(2)-amino
tetrazolyl-(5)-amino or
1,2,4-triazolyl-(3)-amino.

Example 2:

A) Dispersion of the Fluorescent Brightener.

6 g of the fluorescent brightener having the formula:

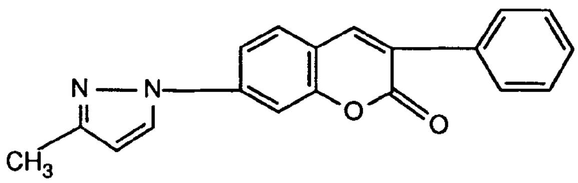

(7)

are ground with 1.5 g of a dispersing agent (condensation product of ditolylethersulfonic acid with formaldehyde-Baykanol SL) and 22.5 g water, over a period of 16 hours, in a glass bead mill. The resulting fine dispersion, containing 20 % fluorescent brightening agent, is then separated from the glass beads in a sieve.

B) Preparation of the Coating Composition

Coating compositions are prepared from the dispersion of the fluorescent brightener of formula (7) in the manner described in part (B) of Example 1.

C) The Paper Coating Application

Commercial base coated paper of LWC quality is coated in a Dow laboratory coater in the manner described in part (C) of Example 1.

The results obtained are as follows:

Table 2

| % Content of Fluorescent Whitener (Active Substance) | | | | | | | | | | | |
|---|---|---|---|---|---|---|---|---|---|---|---|
| 0 | | 0.05 | | 0.10 | | 0.20 | | 0.40 | | 0.80 | |
| $R_{457}$ Whiteness Level | | | | | | | | | | | |
| 77.6 | | 79.5 | | 81.7 | | 82.9 | | 84.4 | | 85.2 | |
| Bleeding Fastness to Water after 10 min/24 h | | | | | | | | | | | |
| 5 | 5 | 4 | 4 | 4 | 4 | 3 | 3 | 4 | 3 | 3 | 3 |

Bleed Fastness is determined according to DIN 53991 - a mark of 5 corresponds to zero bleed, a mark of 1 denotes very severe bleeding.

Example 3:

A) Dispersion of the Fluorescent Brightener.

6 g of the fluorescent brightener having the formula:

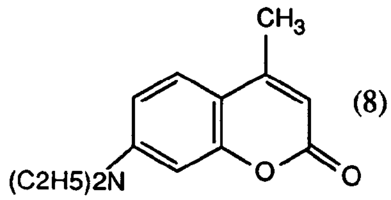

(8)

are ground with 1.5 g of a dispersing agent (condensation product of ditolylethersulfonic acid with formaldehyde-Baykanol SL) and 22.5 g water, over a period of 16 hours, in a glass bead mill. The resulting fine dispersion, containing 20 % fluorescent brightening agent, is then separated from the glass beads in a sieve.

B) Preparation of the Coating Composition

Coating compositions are prepared from the dispersion of the fluorescent brightener of formula (8) in the manner described in part (B) of Example 1.

C) The Paper Coating Application

Commercial base coated paper of LWC quality is coated in a Dow laboratory coater in the manner described in part (C) of Example 1.

The results obtained are as follows:

Table 3

| % Content of Fluorescent Whitener (Active Substance) | | | | | | | | | | | |
|---|---|---|---|---|---|---|---|---|---|---|---|
| 0 | | 0.05 | | 0.10 | | 0.20 | | 0.40 | | 0.80 | |
| $R_{457}$ Whiteness Level | | | | | | | | | | | |
| 77.6 | | 80.8 | | 83.0 | | 86.4 | | 89.5 | | 91.7 | |
| Bleeding Fastness to Water after 10 min/24 h | | | | | | | | | | | |
| 5 | 5 | 4 | 4 | 4 | 4 | 4 | 4 | 4 | 4 | 3 | 3 |

Bleed Fastness is determined according to DIN 53991 - a mark of 5 corresponds to zero bleed, a mark of 1 denotes very severe bleeding.

**Claims**

1. A method for the fluorescent whitening of paper surfaces comprising contacting the paper surface with an aqueous coating composition containing a coumarin-based fluorescent whitening agent.

2. A method according to claim 1 in which the whitening agent has the formula:

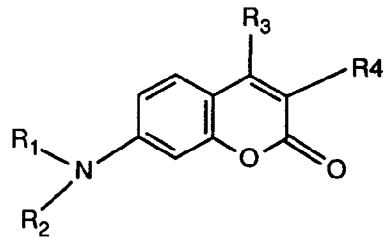

(1)

in which $R_1$ and $R_2$, independently, are hydrogen or $C_1$-$C_4$alkyl or $R_1$ and $R_2$, together with the nitrogen atom to which they are each attached, are the atoms required to complete a pyrazole or triazine ring; $R_3$ is hydrogen or $C_1$-$C_4$alkyl; and $R_4$ is hydrogen or a residue having the formula:

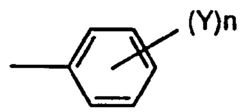

in which n is 1 or 2 and Y is hydrogen, $C_1$-$C_4$alkyl, halogen, $SO_3H$, $SO_2NH_2$, $SO_2NH(C_1$-$C_4$alkyl), $SO_2N(C_1$-$C_4$alkyl$)_2$, $SO_2NHCH_2CH_2OH$, $SO_2NHCH_2CH_2O(C_1$-$C_4$alkyl) or $SO_2NHCH_2CH_2CH_2N(C_1$-$C_4$alkyl$)_2$.

**3.** A method according to claim 2 in which the whitening agent has the formula:

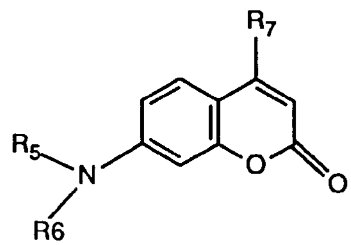

(2)

in which $R_5$, $R_5$ and $R_7$, independently, are $C_1$-$C_4$alkyl.

**4.** A method according to claim 3 in which $R_5$ and $R_6$ are each ethyl and $R_7$ is methyl.

**5.** A method according to claim 1 in which the whitening agent has the formula:

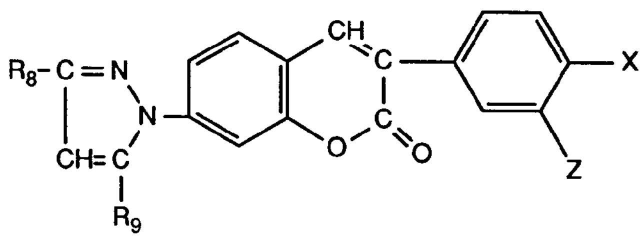

(3)

in which $R_5$ is hydrogen, $C_1$-$C_4$alkyl, phenyl or benzyl; $R_9$ is hydrogen, $C_1$-$C_4$alkyl or phenyl; X is hydrogen, chlorine or $C_1$-$C_4$alkyl; and Z is hydrogen, $-SO_3H$, $-SO_2NH_2$, $-SO_2NH-CH_3$, $-SO_2-NH-CH_2CH_3$, $-SO_2-NH-CH_2CH_2OH$, $-SO_2-NH-CH_2CH_2OCH_3$, $-SO_2-NH-CH_2CH_2-OCH_2CH_3$, $-SO_2-NH-CH_2CH_2N(CH_2CH_3)_2$, $-SO_2-NH-CH_2CH_2CH_2N(CH_3)_2$, $-SO_2-N(CH_3)_2$ or $-SO_2N(CH_2CH_3)_2$.

**6.** A method according to claim 5 in which $R_8$ is hydrogen, methyl or ethyl; $R_9$ is hydrogen or methyl; X is hydrogen, methyl or ethyl; and Z is hydrogen.

**7.** A method according to claim 1 in which the whitening agent is a whitening agent having the formula:

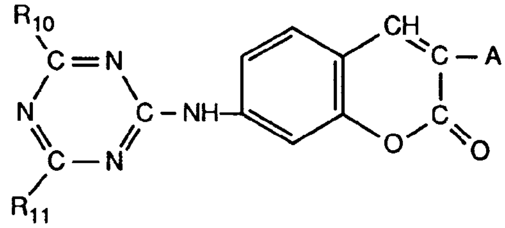

(4)

in which $R_{10}$ is amino, $C_1$-$C_4$alkylamino, di($C_1$-$C_4$alkyl)amino, cyclohexylamino, monoethanolamino, diethanolamino, N-phenylamino, morpholino, piperidino, Cl, Br, hydroxy, $C_1$-$C_4$alkoxy, phenoxy, $C_1$-$C_4$alkylphenoxy, chlorophenoxy, phenylthio, chlorophenylthio, hydroxycyclohexylamino, $C_1$-$C_4$alkylamino-$C_1$-$C_4$alkylamino, N-$C_1$-$C_4$alkylphenylamino, N-$C_1$-$C_4$alkoxyphenylamino, N-chlorophenylamino, benzylamino, N-pyridylamino, N-thiazolylamino, N-dihydro-imidazolylamino, N-triazolylamino or N-tetrazolylamino; $R_{11}$ is amino, $C_1$-$C_4$alkylamino, di-($C_1$-$C_4$-alkyl)amino, cyclohexylamino, monoethanolamino, diethanolamino, N-phenylamino, morpholino or piperidino; and A is phenyl, chlorophenyl, methylphenyl or dimethylphenyl.

8. A method according to claim 7 in which $R_{10}$ is amino, diethylamino, chloro, morpholino, monoethanolamino or N-ethyl-N-butylamino; and $R_{11}$ is diethylamino, monoethanolamino, morpholino, N-ethyl-N-butylamino or phenylthio.

9. A method according to claim 8 in which the whitening agent has the formula:

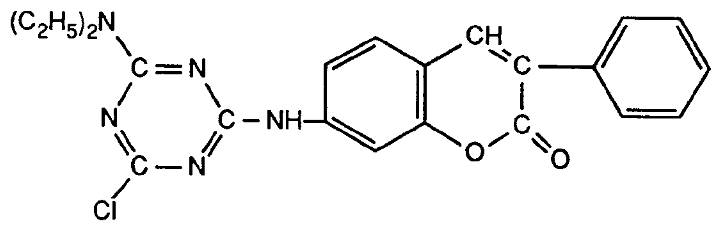

(5) .

10. A method according to any of the preceding claims in which the fluorescent whitener is formulated as an aqueous dispersion or emulsion which contains, in addition to the coumarin-based whitening agent and water, at least one dispersing agent and/or emulsifier and optionally other auxiliaries.

11. A method according to claim 10 in which the aqueous dispersion or emulsion contains customary anionic or cationic and/or nonionic emulsifiers and/or dispersing agents as the dispersing agents and/or emulsifiers.

12. A method according to claim 11 in which the dispersing agents and/or emulsifiers are present in amounts of 2 to 20 % by weight based on the fluorescent brightener.

13. A method according to any of claims 10 to 12 in which the dispersing agent is an anionic dispersing agent.

14. A method according to claim 13 in which the anionic dispersing agent is a naphthalene sulfonic acid/formaldehyde or a ditolylethersulfonic acid/formaldehyde condensation product.

15. A method according to any of claims 10 to 14 in which the dispersion or emulsion also contains 45 to 95 % by weight of water, and optionally preservatives and foam suppressants.

16. A method according to any of claims 10 to 15 in which the auxiliary is a water-insoluble aromatic or $C_{14}$-$C_{18}$ fatty alcohol auxiliary, or auxiliary mixture having a melting point between above 40°C and 200°C.

17. A method according to any of the preceding claims in which the aqueous coating composition is a pigmented coating composition containing an inorganic or organic pigment.

18. A method according to claim 17 in which the pigment is one or more of aluminium silicate, calcium carbonate (clay), barium sulfate, satin white, titanium dioxide, magnesium silicate or a urea-formaldehyde condensate.

**19.** A method according to any of the preceding claims in which the aqueous coating composition contains, as binder, styrene/butyl acrylate, a styrene/butadiene/acrylic acid copolymer or a styrene/butadiene rubber.

**20.** A method according to any of the preceding claims in which the aqueous coating composition contains 30 to 70 % by weight of a white pigment, 1 to 30 % by weight of a binder, based on the weight of the white pigment; and 0.01 to 2 % by weight of the coumarin fluorescent brightener, based on the weight of the white pigment.

**21.** A method according to claim 20 in which the aqueous coating composition contains 30 to 70 % by weight of a white pigment; 5 to 25 % by weight of a binder, based on the weight of the white pigment; and 0.1 to 1 % by weight of the coumarin fluorescent brightener, based on the weight of the white pigment.

**22.** A method according to any of the preceding claims in which the aqueous coating composition contains one or more auxiliaries which function to regulate the rheological properties of the coating composition.

**23.** A method according to claim 22 in which the auxiliary is polyvinyl alcohol or polyvinyl pyrrolidone.

**24.** A method according to any of the preceding claims in which the surfaces which are whitened are of paper, cardboard or photopaper.

**25.** An aqueous coating composition comprising a binder, a coumarin fluorescent brightener and optionally an inorganic or organic pigment.

European Patent Office

# EUROPEAN SEARCH REPORT

Application Number
EP 94 81 0042

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 306 447 (CIBA-GEIGY) * claims 1-14 * ----- | 1 | D21H21/30 D21H19/46 |
| | | | TECHNICAL FIELDS SEARCHED (Int.Cl.5) |
| | | | D21H D06L |

The present search report has been drawn up for all claims

| Place of search | Date of completion of the search | Examiner |
|---|---|---|
| THE HAGUE | 17 May 1994 | Fouquier, J-P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)